Europäisches Patentamt

(19) European Patent Office

Office européen des brevets

(11) Publication number: **0 136 375 B1**

# (12) EUROPEAN PATENT SPECIFICATION

(45) Date of publication of patent specification: **06.11.91**

(51) Int. Cl.⁵: **C12N 5/00, C12P 21/00, A61K 37/02**

(21) Application number: **83304302.9**

(22) Date of filing: **25.07.83**

(54) **A regulatory glycoprotein for immune response in the production of T-cell growth factor.**

(43) Date of publication of application:
**10.04.85 Bulletin 85/15**

(45) Publication of the grant of the patent:
**06.11.91 Bulletin 91/45**

(84) Designated Contracting States:
**AT BE CH DE FR GB IT LI NL SE**

(56) References cited:
**US-A- 4 390 623**
**US-A- 4 406 830**

**Biological abstracts/RRM vol. 26, 1983, ref. 23882. R. Stahn et al "Human T lymphocite activation depends on the presence of a co mitogenic serum glyco protein" & Abstracts of the 15th international leukocyte culture conference, Asilomar, Dec. 5-10-1982, Immunobiol. 1932, vol. 163, n 2-4, page 188**

**Biological abstracts, vol. 73, 1982, ref. 32184. E. Koettgen et al "1-lymphocyte activation studies on the function of mediator proteins" & Klin Wochenschr. 59(12), 669-674, 1981**

**Biological abstracts, vol. 76, 1983, ref. 26579. H.A. Fabricius et al "A comitogenic serum glyc oprotein in T lymphocyte activation" & Immunology 163(5), 436-449, 1982**

**Biological abstracts, vol. 72, 1981, ref. 2939. A.L. Maizel et al "Effect of interleukis 1 on human thymocytes and purified human T cells" & J EXP MED 153(2), 470-475, 1981**

(73) Proprietor: **SHANKSVILLE CORPORATION N.V.**
**Handelskade 8**
**Curacao Netherlands Antilles(NL)**

(72) Inventor: **Fabricius, Hans-Ake**
**Tunibergweg 11**
**W-7814 Breisach(DE)**
Inventor: **Eckart, Ulrich Kottgen**
**Erwinstrasse 39**
**W-7800 Freiburg(DE)**

(74) Representative: **Dixon, Donald Cossar et al**
**Gee & Co. Chancery House Chancery Lane**
**London WC2A 1QU(GB)**

## Description

This invention relates to a process for the production of a serum-free and mitogen-free Interleukin-1 preparation (I1-1) (also known as lymphocyte activating factor LAF).

It has been demonstrated in the literature and also shown in the data reported in co-pending EP-A 0049611, that tumor patients are defective in the ability of their cellular immune system (cell mediated immunity) to product the T-cell growth factor (I1-2) necessary for production of natural killer cells which can attack and destroy tumors. Therefore, if the natural killer cells in tumor patients can be stimulated into activity, it should be possible to stop the growth of tumor cells.

According to current theory of the cell-mediated immunological response mechanism to invasion of the host by a mitogen, the mitogen activates a macrophage subpopulation of lymphocytes to produce I1-1 which in turn stimulates a T-cell subpopulation, presumably T-helper cells, to produce I1-2. The latter is identified biologically by its ability to support the growth of permanently growing T-cell lines.

Production of I1-2, however, depends not only on the interaction of T-lymphocytes with I1-1 but also a protein found in blood plasma sera from humans as well as from other mammals, such as horse sera and bovine sera. Since this protein was originally found in human blood sera, it has been given the acronym PHILIP; Plasmatic Human Interleukin Inducing Protein. It can be characterised by its physioco-chemical properties:- molecular weight of about 90,000 D; glycoprotein; at least one sialic acid residue; strong adherence (binding) to lectins such as wheat germ agglutinin, and Blue A; and relative heat stability: and by its biological activity:- in the presence of Interleukin-1 (serum-free and mitogen-free) it will induce peripheral mononuclear blood cells (lymphocytes: T-cells) to produce T-cell growth factor (I1-2) which forms T-cell blasts and supports the growth of T-cell lines; alternatively, in a serum-free system, in the presence of mitogen such as PHA and peripheral mononuclear blood cells, PHILIP will induce the production of I1-2 and T-cell blastogenesis. In the latter case, the PHA first induces the pro-macrophage cells to produce I1-1 which, with the coaction of PHILIP, induces a second cell fraction, presumably T-helper cells, to produce I1-2.

At this point, it should be emphasised that the mechanism by which PHILIP functions to cooperate with I1-1 and the T-cells to produce I1-2 has not been elucidated. It is generally believed, but has not yet been proven conclusively, that the subpopulation of T-cells known as T-helper cells produce I1-2.

The production of an I1-1 preparation which is serum-free and mitogen-free is therefore highly desirable. Such an I1-1 preparation can be used directly, or after being concentrated (which is made possible by being serum-free), for the production of I1-2.

According to the present invention we provide a process for producing a serum-free and mitogen-free interleukin-1 preparation comprising:

(a) incubating isolated peripheral mononuclear blood cells in a serum-free liquid tissue culture medium to remove serum proteins adhering to the cell membranes;

(b) contacting the incubated blood cells with a mitogen;

(c) washing the activated cells with a sterile liquid to remove the mitogen; and,

(d) incubating the activated cells in a serum-free liquid tissue culture medium to collect interleukin-1 in the liquid phase of the tissue culture medium to thereby obtain a serum-free and mitogen-free interleukin-1, characterised in that the incubated blood cells in said step (b) are contacted for a period of 4 to 8 hours to activate the monocytic cell population of the mononuclear blood cells for the production of interleukin-1.

The serum-free, mitogen-free I1-1 can be prepared from isolated adherent (pro-macrophage) cells or directly from peripheral mononuclear blood cells (PBL). TO isolate the adherent (pro-macrophage) cells, the following well known technique can be used. Peripheral mononuclear blood cells are first isolated from the blood by the density gradient technique. For example, the blood is layered into a tube containing a high density polymeric solution, such as the commercially available Ficoll-hypaque, and centrifuged. The PBL cells are collected above the polymeric solution and carefully removed. The Ficoll-hypaque solution is slightly toxic, so the removal must be done very carefully. The so-obtained PBK cells are washed several times, usually three times, with a sterile solution, to ensure complete removal of the Ficoll-hypaque solution. The washing is accomplished by gently suspending the cells in the sterile solution and centrifuging to again separate the cells from the solution. Low centrifuge speed and time (e.g. 300 x G for ten minutes) are used to avoid breakage or destruction of the cells. Roswell Park Memorial Institute (RPMI) 1640 is a commercially available sterile solution which can be used for this purpose, and also as the liquid culture medium. Machines are presently available for the automatic separation of mononuclear cells from blood, and such machines are advantageously used when available.

The PBL cells are then placed in plastic Petri dishes and incubated for about 1 hour at about 37°C. The pro-macrophage cells are adherent to plastic and adhere to the walls of the Petri dishes. The non-adherent cells are washed off by spraying with sterile solution, e.g. RPMI 1640. The adherent cells are removed from the Petri dish, and a rubber policeman can conveniently be used for this purpose.

The recovered pro-macrophage cells, after being counted (usually about $3 \times 10^6$ cells per ml are used), are incubated for from about 18 to 24 hours at about 37°C using a non-supplemented liquid culture medium, such as RPMI 1640. Fresh culture medium can be added to or replace used culture medium every few hours if desired. By "non-supplemented" is meant that no mitogenic substances or blood serum is added to the culture. All incubation steps in this invention are carried out under a $CO_2$ (about 5-15%) atmosphere, and at a temperature of about 37°C, unless otherwise noted.

The purpose of this preliminary incubation step is to remove any serum proteins which may have been present on the cell membrane surfaces. Since the cell membranes are regenerated about once every eight hours the membranes will "turn over" about 2 or 3 times during this incubation period.

When using the PBL cells without separation of the adherent pro-macrophage cells, the PBL cells can be used directly incubated under the same conditions as described above to remove any proteins which may be present on the surface (membrane) of the cells. The cells are now ready to be activated for the production of II-1.

Activation of the cells is accomplished by contacting the cleaned cells in the liquid culture medium with a mitogen. Phytohemagglutinin A (PHA) is preferably used for this purpose although other lectin type mitogens, such as concanavalin A (Con A), Escherichia coli lipopolysaccharide (LPS) and pokeweedmitogen (PWM) can also be used. An important feature of the invention is that the mitogen is only left in contact with the cells for a short period of time, preferably 4 hours although longer times up to 8 hours can be used. Since the cells are activated or stimulated for and actually begin production of II-1 during this short time period, the mitogen is removed from the cells without significantly limiting the production of II-1.

The washing can be accomplished as described above for removing the Ficoll-hypaque solution, i.e. centrifuging the cell suspension, adding the cells to fresh sterile solution, and repeating this process about 3 times. The supernatants can be saved for future cell stimulations or for recovery of II-1 or PHA.

The activated cells can then be conditioned by incubating the cells in a fresh non-supplemented RPMI 1640 for about 12 to 30 hours, preferably about 15 to 26 hours and most preferably about 14 to 24 hours. Generally, the cells are used in a concentration of about $3 \times 10^6$ cells per ml of culture medium.

A serum-free lectin-free II-1 is obtained in the supernatant. The presence of II-1 is confirmed by known chemical and biological characteristics of Interleukin-1. Thus, the product has been shown by the results of contacting with protease and lectins to be a non-clyco group (i.e. sugar-free) protein, and by gel filtration chromatograph to have a molecular weight of approximately 15,000. The biological characteristics of the II-1 preparation are also consistent with the definition of II-1:

- in the presence of blood serum T-cell blastogenesis is induced; and,
- it is not able to support the growth of a permanently growing T-cell line.

In the present invention, biological activity is determined by a standardized bioassay test which is carried out as follows:

Peripheral mononuclear blood cells which have been isolated and subjected to preliminary incubation to remove serum protein and macrophage cells adhered to the surface of the lymphocyte cells, as described above, are suspended in RPMI 1640 to provide a cell density of one million cells per ml of culture medium.

The supernatant whose biological activity is being assayed is deposited in the first row of a microtiter plate. The second row of wells is filled with RPMI to provide a concentration which is one half that of the first row. Similarly, the third and each succeeding row are each filled with supernatant diluted to a concentration which is one-half of the preceding row. As a result, a microtiter plate is obtained wherein a dilution factor of 1:2 is obtained as one moves from the top towards the bottom of the plate.

A measured number of the mononuclear cells which have been preliminarily incubated is added to each well in the microtiter plate. The growth of the cells as a result of stimulation by T-cell growth factor (II-2) is monitored. In the case of bioassay for II-1 it is further necessary to add serum or PHILIP to each well, since otherwise II-2 production will not occur. Generally, serum is used from a human serum pool in an amount of 15% vol/vol. Likewise, in the bioassay for the biological activity of PHILIP, either Mitogen (PHA) or Interleukin-1 (serum-free) must be added to each well containing the isolated peripheral mononuclear blood cells.

Significant activity is considered to be present in those titer rows where cell density has doubled at the end of seven days. The inverse of dilution for those rows is used as the measure of significant growth.

Example 1

Production of an Interleukin-1 preparation free of mitogen and serum proteins.

a) Preparation by use of adherent pro-macrophages:

Blood lymphocytes are isolated as described in Example 1 of the above mentioned Patent Applications No. 81304560.6. After washing with sterile culture medium, the cells, usually 400 to 800 millions isolated per 400 ml whole blood, are incubated for 60 minutes at 37°C in RPMI 1640 under standard cell culture conditions. The incubation is performed on a plastic Petri dish with a diameter of 15 cm. Such dishes are commercially readily available. After incubation, the cell-containing medium is poured off the dish and the dish with the adhering cells, usually 10% of the total cell number, is rinsed several times, usually 6, with RPMI 1640 to remove all non-adherent cells. Thereafter, the adherent cells are removed from the dish by use of a rubber policeman, counted, and suspended in a concentration of 3 millions/ml in RPMI 1640, and incubated for 24 hours at 37°C under standard cell culture conditions before further processing. After incubation, the cells are stimulated with PHA for 4 to 8 hours and preferably 4 hours, at a concentration of 4 μg PHA/ml of culture medium. The PHA is then discarded, and the cells washed 3 times as described above, whereafter the cells are incubated in RPMI 1640 without supplements for 18 - 24 hours, preferably 20 hours. The culture supernatants are collected, whereafter the cells can be stimulated again with PHA if so desired. The stimulation may be repeated up to 3 times if so desired. The serum free culture supernatants can be concentrated on a YM 10 membrane as des-in Example 1 of the Patent Application No.81304560.6, if desired. The Il-1 containing supernatants can be stored at -20° until they are used.

b) Preparation by use of isolated blood lymphocytes without separation of adherent macrophages:

Blood lymphocytes are isolated as described above in this Example. The cells, after washing with RPMI 1640, are incubated at 37°C under standard cell culture conditions for 24 hours. Thereafter, the cells are counted and stimulated with PHA at a concentration of 4 μg/ml culture medium and a cell density of 3 millions/ml as described above in this Example. All remaining steps of the preparation of Il-1 are the same as those mentioned above in this Example.

Example 2

Biological test for Interleukin-1 activity.

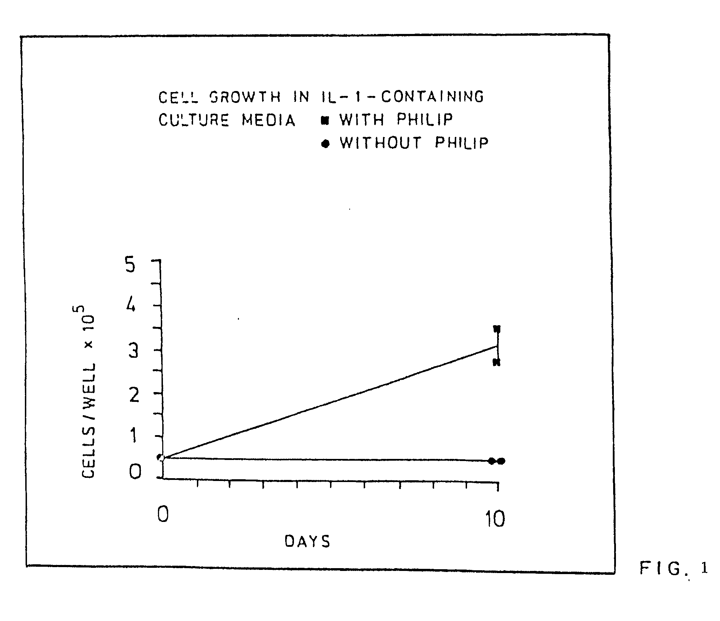

Interleukin-1, free of lectin and sera, prepared as described in Example 1 is filtered sterile and tested for its biologic activity as follows:
RPMI 1640 is mixed with either 15% inactivated human AB serum or with PHILIP to a titer of 8 in the end volume. 200 μl thereof is introduced into the top row of wells in a microtiter plate. In the 4 following rows, 100 ml of the mixture is applied to each well. 200 μl of the Il-1 is added to the wells of the top row and mixed with the RPMI 1640 supplemented by serum PHILIP. 100 μl is transferred to the next row, mixed, and so on. Thus, a serial dilution of Il-1 in dilution steps of 1:2 is achieved. 20 to 50 μl of isolated blood lymphocytes as described in Example 1 of the Patent Application No. 81304560.6 are added. As a consequence of the interaction of Il-1 and PHILIP, the cells start synthesizing Il-2. The effectiveness of the synthesis is dependent on the concentration of Il-1. Il-1 activity is quantificated by the titer it exhibits in the culture (i.e. by the inverse of the dilution in which the cell number has doubled within seven days in culture) - see Figure 1.

**Claims**

1. A process for producing a serum-free and mitogen-free interleukin-1 preparation comprising:
   (a) incubating isolated peripheral mononuclear blood cells in a serum-free liquid tissue culture medium to remove serum proteins adhering to the cell membranes;
   (b) contacting the incubated blood cells with a mitogen
   (c) washing the activated cells with a sterile liquid to remove the mitogen; and,
   (d) incubating the activated cells in a serum-free liquid tissue culture medium to collect interleukin-1 in the liquid phase of the tissue culture medium to thereby obtain a serum-free and mitogen-free interleukin-1, characterised in that the incubated blood cells in said step (b) are contacted for a period of 4 to 8 hours to activate the monocytic cell population of the mononuclear blood cells for the production of interleukin-1.

2. A process as claimed in Claim 1, wherein the isolated peripheral mononuclear blood cells in said step (a) are incubated for a period of 18 to 24 hours and wherein in said step (d) the activated cells are incubated for 18 to 24 hours.

3. A process as claimed in Claim 1 or 2, wherein the isolated peripheral mononuclear blood cells used in said step (a) are isolated pro-macrophage cells separated from peripheral mononuclear blood cells and wherein the mitogen used in said step (b) is preferably phytohemagglutinin A and the incubated calls are left in contact with the mitogen for 4 hours.

**Revendications**

1. Procédé pour produire une préparation d'interleukine-1 exempte de sérum et de mitogène, comprenant les étapes consistant à :
   (a) incuber des cellules mononucléées isolées du sang périphérique dans un milieu de culture de tissus liquide exempt de sérum, afin d'enlever les protéines sériques adhérant aux membranes cellulaires;
   (b) mettre en contact les cellules du sang incubées avec un mitogène;
   (c) laver les cellules activées avec un liquide stérile pour enlever le mitogène; et à
   (d) incuber les cellules activées dans un milieu de culture de tissus liquide exempt de sérum, pour recueillir l'interleukine-1 dans la phase liquide du milieu de culture de tissus, grâce à quoi on obtient une interleukine-1 exempte de sérum et de mitogène, caractérisé en ce que les cellules du sang incubées dans ladite étape (b) sont mises en contact pendant une durée de 4 à 8 heures pour activer la fraction de cellules monocytaires des cellules mononucléées du sang pour la production de l'interleukine-1.

2. Procédé selon la revendication 1, où les cellules mononucléées isolées du sang périphérique sont incubées dans l'étape (a) pendant une durée de 18 à 24 heures et où dans ladite étape (d) les cellules activées sont incubées pendant 18 à 24 heures.

3. Procédé selon la revendication 1 ou 2, où les cellules mononucléées isolées du sang périphérique utilisées à ladite étape (a) sont des cellules de pro-macrophages séparées des cellules mononucléées du sang périphérique, où le mitogène utilisé à ladite étape (b) est de préférence la phyto-hémagglutinine A et où les cellules incubées sont laissées en contact avec le mitogène pendant 4 heures.

**Patentansprüche**

1. Verfahren zur Herstellung eines serum- und mitogen-freien Interleukin-1-Präparats, bestehend aus den Schritten:
   (a) Beschicken eines Inkubators mit isolierten, peripheren, mononuklearen Blutzellen in einem serumfreien, flüssigen, Gewebekulturenmedium zum Entfernen der an den Zellmembranen haftenden Serumproteine;
   (b) In Kontakt bringen der Blutserumzellen, mit denen der Inkubator beschickt wurde, mit einem Mitogen;
   (c) Waschen der aktivierten Zellen mit einer sterilen Flüssigkeit zum Entfernen des Mitogens; und
   (d) einen Inkubator mit den aktivierten Zellen in einem serumfreien, flüssigen Gewebekulturmedium beschicken, um Interleukin-1 in der flüssigen Phase des Gewebekulturenmediums zu gewinnen zwecks Erhalt von serum- und mitogenfreien Interleukins-1, dadurch gekennzeichnet, dass die Blutzellen, mit denen der Inkubator beschickt wurde in obigem Schritt (b) während 4 bis 8 Stunden in Kontakt gebracht werden, um die monozytische Zellenpopulation der mononuklearen Blutzellen zur Herstellung von Interleukin-1 zu aktivieren.

2. Verfahren nach Patentanspruch 1, worin isolierte, periphere, mononukleare Blutzellen im obigen Schritt (a) während eines Zeitraums von 18 bis 24 Stunden in einen Inkubator gegeben werden, und wobei im Schritt (d) die aktivierten Zellen während 18 bis 24 Stunden in einen Inkubator gegeben werden.

3. Verfahren nach Patentanspruch 1 oder 2, worin die im obigen Schritt (a) verwendeten, isolierten, peripheren, mononuklearen Zellen isolierte Prophagozytenzellen sind, die aus peripheren, mononuklearen Blutzellen separiert werden, und worin das im obigen Schritt (b) verwendete Mitogen vorzugsweise Phyto-Hämagglutin A ist, und die Zellen, mit denen der Inkubator beschickt wurde, darin während 4 Stunden mit dem Mitogen in Kontakt belassen werden.

CELL GROWTH IN IL-1-CONTAINING
CULTURE MEDIA ■ WITH PHILIP
● WITHOUT PHILIP

FIG. 1

EP 0 136 375 B1